(19) Europäisches Patentamt
European Patent Office
Office européen des brevets

(11) **EP 1 494 726 B1**

(12) **FASCICULE DE BREVET EUROPEEN**

(45) Date de publication et mention
de la délivrance du brevet:
**31.12.2008   Bulletin 2009/01**

(51) Int Cl.:
*A61L 15/46* (2006.01)     *A01N 59/16* (2006.01)
*A01N 37/36* (2006.01)

(21) Numéro de dépôt: 03746346.0

(22) Date de dépôt: **15.04.2003**

(86) Numéro de dépôt international:
**PCT/FR2003/001194**

(87) Numéro de publication internationale:
**WO 2003/086492 (23.10.2003 Gazette 2003/43)**

(54) **UTILISATION DE SELS METALLIQUES DU GLUCONATE POUR LA FABRICATION DE SUBSTRATS A ACTIVITE ANTIMICROBIENNE**

VERWENDUNG VON METALLGLUCONATSALZEN BEI DER HERSTELLUNG VON ANTIMIKROBIELL WIRKSAMEN SUBSTRATEN

USE OF METALLIC GLUCONATE SALTS IN THE PRODUCTION OF ANTIMICROBIALLY ACTIVE SUBSTRATES

(84) Etats contractants désignés:
**AT BE BG CH CY CZ DE DK EE ES FI FR GB GR HU IE IT LI LU MC NL PT RO SE SI SK TR**

(30) Priorité:  **15.04.2002   FR 0204665**

(43) Date de publication de la demande:
**12.01.2005   Bulletin 2005/02**

(73) Titulaire: **Georgia-Pacific France**
**68320 Kunheim (FR)**

(72) Inventeurs:
• **BRET, Bruno**
**F-68920 WINTZENHEIM (FR)**

• **ROUSSIN-MOYNIER, Yves**
**F-68920 WINTZENHEIM (FR)**
• **BOURGEOIS, Michel**
**F-69003 LYON (FR)**
• **NORMAND, Xavier**
**F-69770 MONTROTTIER (FR)**

(74) Mandataire: **Giraud, Françoise et al**
**Cabinet Beau de Loménie**
**158, rue de l'Université**
**75340 Paris Cedex 07 (FR)**

(56) Documents cités:
WO-A-98/26808          DE-A- 3 443 985
US-A1- 2003 096 545

**Description**

L'invention a pour objet l'utilisation de sels métalliques particuliers pour la fabrication de substrats, à base de fibres notamment cellulosiques, à activité antimicrobienne, notamment antibactérienne et antifongique.

L'invention trouve notamment application dans le domaine sanitaire, hygiénique et alimentaire.

Le brevet EP-B-113 254 décrit un non-tissé comprenant une nappe de fibres textiles, un liant à base de polymère pour lier ensemble ces fibres, et une faible quantité d'un agent antimicrobien incorporée dans ce liant ; ledit agent antimicrobien étant avantageusement choisi parmi les nitriles aromatiques halogénés, le sulfate d'imazalile, le 3,5,3', 4'-tétrachlorosalicylanilide ou l'hexachlorophène.

Le brevet EP-B-431 002 décrit un tissu pour la désinfection ou le blanchiment, qui comprend une première et une seconde couches de substrat liées ensemble avec un polymère adhésif et entre lesquelles sont retenues des particules solides, lesdites particules comprenant un agent libérant du chlore.

La demande de brevet WO-A-01 32138 concerne l'utilisation d'un agent antimicrobien pour la fabrication d'un article d'essuyage jetable pour réduire le nombre de microbes transférés vers la main lorsqu'on essuie une surface avec ledit article. L'agent antimicrobien est choisi parmi les composés phénoliques, isothiazolinone, pyrazole ou ammonium quaternaire, les agents oxydants, les quinoléines, les guanidines ou les aldéhydes.

Par ailleurs, on connaît les propriétés antiseptiques ou comme source d'apport ou supplément, du gluconate de zinc, du gluconate de cuivre et du gluconate d'argent.

Il a maintenant été trouvé de manière inattendue, et c'est le fondement de la présente invention, que des substrats comprenant certains sels métalliques du gluconate possèdent une activité antimicrobienne.

Ainsi, l'invention a pour objet l'utilisation du gluconate de zinc, d'argent ou de cuivre comme agent antimicrobien, notamment antibactérien et antifongique, pour la fabrication de substrats à base de fibres notamment cellulosiques. Le sel de gluconate préféré selon l'invention est le gluconate de zinc.

Selon un premier aspect, l'invention concerne un substrat à base de fibres papetières, de type non tissé, obtenu par voie sèche, caractérisé en ce qu'il comprend un liant thermoplastique permettant aux fibres de se lier entre elles et du gluconate de zinc, d'argent ou de cuivre, à titre d'agent antimicrobien.

Selon un deuxième aspect, l'invention concerne un procédé de préparation de ce substrat selon lequel ledit agent anti-microbien est incorporé dans le substrat par pulvérisation d'un mélange liquide comprenant ledit liant thermoplastique et ledit agent antimicrobien sur le substrat ou par imprégnation ou enduction du substrat avec ledit mélange.

Selon un troisième aspect, l'invention concerne l'utilisation d'un substrat faisant l'objet du premier aspect ou obtenu selon le procédé faisant l'objet du deuxième aspect pour la fabrication d'un article sanitaire, d'un article d'hygiène ou d'un article pour emballage alimentaire.

Les substrats conformes à l'invention sont des non-tissés à base de fibres papetières obtenus par voie sèche.

Les non-tissés sont des feuilles ou nappes de fibres orientées dans une direction ou au hasard et liées par des moyens mécaniques (de friction), des moyens chimiques (apport d'adhésif) ou thermiques.

Le procédé d'obtention des non-tissés à base de fibres papetières par voie sèche consiste, de manière bien connue de l'homme du métier, à traiter des pâtes papetières afin de les défibrer à sec, à former un voile sur une toile de formation où les fibres individualisées sont réparties au hasard par voie éraulique, à apporter un liant thermoplastique qui va pénétrer dans le voile ainsi formé permettant aux fibres de se lier entre elles, puis à sécher et à réticuler. Le liant thermoplastique peut être constitué de latex, comme par exemple un copolymère d'éthylène et d'acétate de vinyle (EVA), ou de fibres thermoliantes. Une feuille de non-tissé obtenue par ce procédé a généralement un grammage d'environ 40 à 120 g/m$^2$.

Conformément à l'invention, le substrat comprend un agent antimicrobien, notamment antibactérien et antifongique, tel que défini ci-dessus.

L'agent antimicrobien peut être incorporé dans le substrat par exemple par pulvérisation d'un mélange liquide liant thermoplastique + agent antimicrobien sur le substrat, ou bien encore par imprégnation ou enduction du substrat avec le mélange précité, ces techniques étant bien connues de l'homme du métier. Lorsque la technique de pulvérisation est mise en oeuvre, la quantité de mélange pulvérisé sur le substrat est généralement comprise entre environ 12 et 24 g/m$^2$.

La concentration en agent antimicrobien dans le produit fini est d'environ 0,01 à 10 % en masse, de préférence d'environ 0,05 à 1 % en masse. Ceci correspond à une concentration en matière sèche de l'agent antimicrobien d'environ 0,006 à 6 g/m$^2$, de préférence d'environ 0,03 à 0,6 g/m$^2$.

Le substrat conforme à l'invention présente les avantages suivants :

- il possède un large spectre d'activité sur les micro-organismes gram-négatifs (par exemple *Pseudomonas aeruginosa*) et sur les micro-organismes gram-positifs (par exemple *Staphylococcus aureus*) ;
- il possède une innocuité alimentaire.

De ce fait, le substrat conforme à l'invention, comprenant un sel métallique de gluconate à titre d'agent anti-microbien, trouve notamment application :

- dans des articles sanitaires, comme essuie-mains, papier toilette, mouchoirs, lingette imprégnée, papier absorbant ;
- dans des articles d'hygiène féminine, par exemple comme composant (matelas absorbant) pour serviette hygiénique, ou pour bébés, comme lingette imprégnée ;
- dans des emballages alimentaires, comme papier absorbant pour barquette à viande.

L'invention sera illustrée à l'aide des exemples et tests suivants. Dans ces exemples et tests, on utilise les abréviations suivantes :

AN = souche *Aspergillus niger* ATCC 16404
CA = souche *Candida albicans ATCC* 10231
EC = souche *Escherichia coli ATCC* 11229
PA = souche *Pseudomonas aeruginosa* ATCC 9027
SA = souche *Staphylococcus aureus* ATCC 6538
(ATCC = American Type Culture Collection)
CMI = Concentration Minimum Inhibitrice
EVA = copolymère d'éthylène et d'acétate de vinyle
UFC = Unité Formant Colonie
ZI = zone d'inhibition

L'activité antimicrobienne des substrats conformes à l'invention est évaluée qualitativement et quantitativement d'après les normes détaillées ci-dessous.

**Evaluation qualitative**

a) Norme suisse SNV 195 920 : Etoffes - Contrôle de l'activité antibactérienne : Test de diffusion dans de l'agar

Des éprouvettes de 25 à 30 mm de diamètre de substrat, traité avec l'agent antimicrobien selon l'invention, sont déposées sur une double couche de gélose nutritive, ensemencée avec les bactéries tests et l'ensemble est incubé pendant 18 h/24 h à 37˚C.
Ensuite, la zone d'inhibition autour de l'éprouvette est mesurée et se calcule en divisant par 2 la différence entre le diamètre total de l'éprouvette augmentée de la zone d'inhibition et le diamètre de l'éprouvette.
L'éprouvette est retirée de la zone de contact observée en appréciant le développement bactérien permettant de différencier plusieurs niveaux d'efficacité.
Les souches utilisées dans ce test sont les suivantes:

- *Staphylococcus aureus* ATCC 6538
- *Escherichia coli ATCC* 11229
- *Pseudomonas aeruginosa* ATCC 9027

b) Norme suisse SNV 195 921 : Etoffes - Contrôle de l'activité antifongique : Test de diffusion dans de l'agar

Des éprouvettes de 25 à 30 mm de diamètre de substrat traité sont déposées sur une double couche de gélose nutritive, ensemencées avec les bactéries tests et l'ensemble est incubé.
Ensuite, la zone d'inhibition autour de l'éprouvette est mesurée et se calcule en divisant par 2 la différence entre le diamètre total de l'éprouvette augmentée de la zone d'inhibition et le diamètre de l'éprouvette.
L'éprouvette est retirée de la zone de contact observée en appréciant le développement bactérien permettant de différencier plusieurs niveaux d'efficacité.
Les souches utilisées dans ce test sont les suivantes :

- *Aspergillus niger ATCC* 16404
- *Candida albicans* ATCC 10231

**Evaluation quantitative**

Norme AFNOR XPG 39010 : Propriétés des étoffes - Etoffes et surfaces polymériques à propriétés antibactériennes - Caractérisation et mesure de l'activité bactériostatique (inoculation des éprouvettes par transfert)

Cette norme permet de déterminer l'activité bactériostatique à la surface des étoffes et des surfaces polymériques agissant par contact ou par diffusion de l'actif antibactérien, que les étoffes soient hydrophiles ou hydrophobes.

L'essai est effectué après un cycle d'entretien ou non (usage unique).

Les échantillons sont lavés afin d'éliminer les traces d'ensimage et d'obtenir un produit hygiéniquement propre.

Les éprouvettes sont déposées sur la surface gélosée d'une boîte de Pétri inoculée par inondation avec 1 ml de suspension bactérienne de 1 à $3.10^6$ - UFC/ml.

Le contact substrat-gélose est assuré à l'aide d'un cylindre en acier inoxydable de 200 g pendant 1 minute.

L'éprouvette est déposée dans une boîte de Pétri stérile, face ensemencée vers le haut, et l'ensemble est incubé à 37°C en chambre humide pendant 24 heures ou une semaine.

L'éprouvette est placée dans un sachet stérile. 20 ml de diluant contenant un neutralisant sont ajoutés. L'ensemble est malaxé dans un appareil de type "Stomacher" 1 minute sur chaque côté.

Cette procédure est aussi appliquée à des éprouvettes de coton non traité (servant de témoin).

Expression des résultats

Les concentrations bactériennes sont exprimées en :

- UFC (Unités Formant Colonies),
- log d'UFC,
- différence de log d'UFC :

$$\Delta_{24h} = \log(UFC_{24h}) - \log(UFC_{0h})$$

$$\Delta_{1sem.} = \log(UFC_{1sem.}) - \log(UFC_{0h})$$

La condition pour qu'un substrat soit bactériostatique selon la norme XPG 39010 est :

$$-2 < \Delta_{24h} < +2$$

$$-2 < \Delta_{1sem.} < +2$$

L'efficacité antimicrobienne est meilleure dans la plupart des exemples donnés ci-après.

Plus le $\Delta_{24h}$ ou $\Delta_{1\ sem}$ est irieur à +2, voire inférieur à -2, plus le nombre de bactéries tuées sur le substrat par l'agent antimicrobien est important, et plus le substrat est bactéricide.

Lorsque le nombre d'UFC est proche de zéro ou égal à zéro, le substrat est bactéricide.

**Exemple 1 :** Préparation d'un substrat en non-tissé

On prépare une solution contenant 0,2 g de gluconate de zinc, 9,8 g d'EVA et 9,8 g d'eau. Cette solution est pulvérisée (12 g/m$^2$) sur la face intérieure d'un non-tissé à 120 g/m$^2$, séparé en deux. Ce non-tissé est à base de fibres exclusivement papetières et obtenu par voie sèche en utilisant comme liant de l'EVA. La concentration en gluconate de zinc dans le produit fini est de 0,2 % en masse.

**Exemple 2 :** Préparation d'un substrat en non-tissé

On répète le mode opératoire de l'exemple 1, mais en utilisant un non-tissé à base de fibres exclusivement papetières et obtenu par voie sèche en utilisant comme liant de l'EVA, qui a été imprégné à 300 % d'une lotion standard pour lingettes bébé avant l'étape de pulvérisation.

**Exemple 3 :** Préparation d'un substrat en non-tissé

On répète le mode opératoire de l'exemple 1, mais en utilisant un non-tissé à 120 $g/m^2$ séparé en deux et traité sur une face par de l'EVA. Ce non-tissé est à base de fibres exclusivement papetières et obtenu par voie sèche en utilisant comme liant de l'EVA. La solution de gluconate de zinc et d'EVA telle que décrite à l'exemple 1 est appliquée par pulvérisation sur la face non traitée du non-tissé.

**Exemple 4 :** Préparation d'un substrat en non-tissé

Un non-tissé à 60 $g/m^2$ est traité industriellement par pulvérisation de la solution de gluconate de zinc et d'EVA décrite à l'exemple 1 sur les deux faces. Ce non-tissé est à base de fibres exclusivement papetières et obtenu par voie sèche en utilisant comme liant de l'EVA.

**Test 1 :** Mesures de CMI du gluconate de zinc

Les CMI sont présentées dans le tableau suivant.

Tableau 1

| Souche | SA | PA | EC | CA | AN |
|---|---|---|---|---|---|
| CMI (ppm) | 5000 | 12500 | 6250 | 3 120 | 6250 |

**Test 2 :** Mise en évidence de l'activité antibactérienne et anti-fongique de substrats selon l'invention

On a testé les activités des substrats des exemples 1 et 2 selon les normes suisses SNV 195 920 et, respectivement, SNV 195 921. Les résultats sont présentés dans le Tableau suivant.

Tableau 2

| Souche | EC | PA | CA | AN |
|---|---|---|---|---|
| Exemple 1 | ZI = 0 | ZI = 0 | ZI = 0 | ZI = 0 |
| Exemple 2 | ZI = 0 | ZI = 0 | ZI = 0 | ZI = 0 |

Ces résultats montrent que le gluconate de zinc ne migre pas. Les substrats selon l'invention peuvent donc trouver application notamment dans le domaine alimentaire, par exemple comme papier absorbant pour barquette à viande.

**Test 3 :** Mise en évidence de l'activité antibactérienne d'un substrat selon l'invention

On a testé l'activité du substrat de l'exemple 1 sur les souches *Staphylococcus aureus* ATCC 6538, *Pseudomonas aeruginosa* ATCC 9027 et *Escherichia coli* ATCC 11229 selon la norme AFNOR XPG 39010 en utilisant, comme gélose, de la gélose Columbia (commercialisée par Bio-Mérieux), comprenant 5 % en masse de sang de mouton. Les résultats sont présentés dans les Tableaux suivants.

Tableau 3 (*Staphylococcus aureus*)

| Substrat testé | Log ($UFC_{0h}$) | Log ($UFC_{24h}$) | $\Delta_{24h}$ |
|---|---|---|---|
| Exemple 1 | 4,80 | 0,00 ❶ | -4,80 |

(suite)

| Substrat testé | Log (UFC$_{0h}$) | Log (UFC$_{24h}$) | $\Delta_{24h}$ |
|---|---|---|---|
| Témoin | 4,83 | 8,64 | 3,81 |
| ❶ Lorsque le nombre d'UFC est égal à zéro, arbitrairement le log(UFC) est égal à 0. | | | |

Tableau 4 (*Pseudomonas aeruginosa*)

| Substrat testé | Log (UFC$_{0h}$) | Log (UFC$_{24h}$) | $\Delta_{24h}$ |
|---|---|---|---|
| Exemple 1 | 5,26 | 0,00 ❶ | -5,26 |
| Témoin | 5,19 | 9,69 | 4,50 |
| ❶ Lorsque le nombre d'UFC est égal à zéro, arbitrairement le log(UFC) est égal à 0. | | | |

Tableau 5 (*Escherichia coli*)

| Substrat testé | Log (UFC$_{0h}$) | Log (UFC$_{24h}$) | $\Delta_{24h}$ |
|---|---|---|---|
| Exemple 1 | 5,15 | 0,00 ❶ | -5,15 |
| Témoin | 5,06 | 9,41 | 4,35 |
| ❶ Lorsque le nombre d'UFC est égal à zéro, arbitrairement le log(UFC) est égal à 0. | | | |

**Test 4 :** Mise en évidence de l'activité antibactérienne d'un substrat selon l'invention

On a testé l'activité du substrat de l'exemple 3 sur les souches *Staphylococcus aureus* ATCC 6538 et *Pseudomonas aeruginosa* ATCC 9027 selon la norme AFNOR XPG 39010 en utilisant, comme gélose, de la gélose Columbia comprenant éventuellement 5 % en masse de sang de mouton. Les résultats sont présentés dans les Tableaux suivants.

Tableau 6 (*Staphylococcus aureus,* gélose au sang)

| Substrat testé | Log (UFC$_{0h}$) | Log (UFC$_{24h}$) | $\Delta_{24h}$ |
|---|---|---|---|
| Exemple 3 | 5,11 | 0,00 ❶ | -5,11 |
| Témoin | 5,13 | 8,18 | 3,05 |
| ❶ Lorsque le nombre d'UFC est égal à zéro, arbitrairement le log(UFC) est égal à 0. | | | |

Tableau 7 (*Pseudomonas aeruginosa*)

| Substrat testé | Log (UFC$_{0h}$) | Log (UFC$_{24h}$) | $\Delta_{24h}$ |
|---|---|---|---|
| Exemple 3 | 5,08 | 0,79 | -4,29 |
| Témoin | 4,94 | 9,10 | 4,16 |

Tableau 8 (*Pseudomonas aeruginosa,* gélose au sang)

| Substrat testé | Log (UFC$_{0h}$) | Log (UFC$_{24h}$) | $\Delta_{24h}$ |
|---|---|---|---|
| Exemple 3 | 5,09 | 1,62 | - 3,47 |
| Témoin | 5,02 | 9,64 | 4,62 |

**Test 5 :** Mise en évidence de l'activité antibactérienne d'un substrat selon l'invention.

On a testé l'activité du substrat de l'exemple 4 sur les souches *Staphylococcus aureus* ATCC 6538 et *Pseudomonas aeruginosa* ATCC 9027 selon la norme AFNOR XPG 39010, en utilisant de la gélose Columbia comprenant 5 % en masse de sang de mouton (trois éprouvettes de l'exemple 4 et deux éprouvettes du témoin ont été testées). Les résultats sont présentés dans les Tableaux suivants.

TABLEAU 9 (*Staphylococcus aureus*)

| Temps d'incubation | | 0 h | | | | 24 h | | | | |
|---|---|---|---|---|---|---|---|---|---|---|
| Substrat testé | Eprouvette | UFC | log $(UFC_{oh})$ | Ecart type | Moyenne | UFC | log $(UFC_{24h})$ | Ecart type | Moyenne | $\Delta_{24h}$ (moy.) |
| Exemple 4 | 1 | $9,98.10^4$ | 5,00 | 0,07 | 5,07 | 0,00 | 0,00 ❶ | 1,76 | ❷ | - 5,07 |
| | 2 | $1,34.10^5$ | 5,13 | | | $1,12.10^3$ | 3,05 | | | - 2,02 |
| | 3 | $1,23.10^5$ | 5,09 | | | 0,00 | 0,00 | | | - 5,07 |
| Témoin | 1 | $8,41.10^4$ | 4,93 | 0,12 | 5,01 | $5,24.10^8$ | 8,72 | 0,12 | 8,80 | 3,79 |
| | 2 | $1,25.10^5$ | 5,10 | | | $7,69.10^8$ | 8,89 | | | |

❶    Lorsque le nombre d'UFC est égal à zéro, arbitrairement le log(UFC) est égal à 0.

❷    La moyenne n'a pas été calculée, car la différence des valeurs extrêmes des logarithmes est supérieure à 1.

TABLEAU 10 (*Staphylococcus aureus*)

| Temps d'incubation | | 0 h | | | | 1 semaine | | | | |
|---|---|---|---|---|---|---|---|---|---|---|
| Substrat testé | Eprouvette | UFC | log(UFC$_{0h}$) | Ecart type | Moyenne | UFC | log(UFC$_{1sem.}$) | Ecart type | Moyenne | $\Delta_{1sem. (\mu o \psi.)}$ |
| Exemple 4 | 1 | $9,98.10^4$ | 5,00 | 0,07 | 5,07 | 0,00 | 0,00 | 0,00 | 0,00 | - 5,07 |
| | 2 | $1,34.10^5$ | 5,13 | | | 0,00 | 0,00 ❶ | | | |
| | 3 | $1,23.10^5$ | 5,09 | | | 0,00 | 0,00 | | | |
| Témoin | 1 | $8,41.10^4$ | 4,93 | 0,12 | 5,01 | $5,95.10^7$ | 7,77 | 0,16 | 7,89 | 2,88 |
| | 2 | $1,25.10^5$ | 5,10 | | | $1,01.10^8$ | 8,01 | | | |

❶ Lorsque le nombre d'UFC est égal à zéro, arbitrairement le log(UFC) est égal à 0.

TABLEAU 11 (*Pseudomonas aeruginosa*)

| Temps d'incubation | | 0 h | | | | 24 h | | | | |
|---|---|---|---|---|---|---|---|---|---|---|
| Substrat testé | Eprouvette | UFC | $\log(UFC_{0h})$ | Ecart type | Moyenne | UFC | $\log(UFC_{24h})$ | Ecart type | Moyenne | $\Delta_{24h\ (\mu o\psi.)}$ |
| Exemple 4 | 1 | $1,44.10^5$ | 5,16 | 0,01 | 5,16 | 0,00 | 0,00 | 0,00 | 0,00 | - 5,16 |
| | 2 | $1,47.10^5$ | 5,17 | | | 0,00 | 0,00 ❶ | | | |
| | 3 | $1,43.10^5$ | 5,15 | | | 0,00 | 0,00 | | | |
| Témoin | 1 | $1,41.10^5$ | 5,15 | 0,08 | 5,10 | $3,84.10^9$ | 9,58 | 0,01 | 9,59 | 4,49 |
| | 2 | $1,10.10^5$ | 5,04 | | | $3,95.10^9$ | 9,60 | | | |

❶ Lorsque le nombre d'UFC est égal à zéro, arbitrairement le log(UFC) est égal à 0.

EP 1 494 726 B1

Les résultats des Tableaux 3 à 11 montrent l'excellente activité antibactérienne des substrats conformes à l'invention.

## Revendications

1. Substrat à base de fibres papetières, de type non tissé, obtenu par voie sèche, **caractérisé en ce qu'**il comprend un liant thermoplastique permettant aux fibres de se lier entre elles et du gluconate de zinc, d'argent ou de cuivre, à titre d'agent antimicrobien.

2. Substrat selon la revendication 1, **caractérisé en qu'**il comprend de environ 0,01 à 10 %, de préférence de environ 0,05 à 1 % en masse d'agent antimicrobien.

3. Substrat selon la revendication 1 ou 2, **caractérisé en ce que** ledit agent antimicrobien est le gluconate de zinc.

4. Substrat selon l'une des revendications 1 à 3, **caractérisé en ce que** ledit liant thermoplastique est constitué d'un latex, par exemple d'un copolymère d'éthylène et d'acétate de vinyle (EVA) ou de fibres thermoliantes.

5. Substrat selon l'une des revendications 1 à 4, **caractérisé en ce que** ledit liant thermoplastique est un copolymère d'éthylène et d'acétate de vinyle (EVA).

6. Procédé de préparation d'un substrat selon l'une des revendications 1 à 5, **caractérisé en ce que** ledit agent antimicrobien est incorporé dans le substrat par pulvérisation d'un mélange liquide comprenant ledit liant thermoplastique et ledit agent antimicrobien sur le substrat ou par imprégnation ou enduction du substrat avec ledit mélange.

7. Utilisation d'un substrat tel que défini dans l'une des revendications 1 à 5 ou tel qu'obtenu par le procédé de la revendication 6 pour la fabrication d'un article sanitaire, d'un article d'hygiène ou d'un article pour emballage alimentaire.

8. Utilisation selon la revendication 7, **caractérisée en ce que** ledit substrat est utilisé pour la préparation d'une lingette imprégnée ou d'un papier absorbant.

9. Utilisation selon la revendication 7, **caractérisée en ce que** ledit substrat est un article pour hygiène féminine, tel qu'un matelas absorbant, ou pour bébé, tel qu'une lingette imprégnée.

10. Utilisation selon la revendication 7, **caractérisé en ce que** ledit substrat est utilisé pour la préparation d'un emballage alimentaire, notamment en tant que papier absorbant pour barquettes à viande.

## Claims

1. Substrate of the nonwoven type, based on paper fibers, obtained by the dry method, **characterized in that** it comprises a thermoplastic binder enabling the fibers to bind together and zinc, silver or copper gluconate as an antimicrobial agent.

2. Substrate according to claim 1, **characterized in that** it comprises from about 0.01 to 10%, preferably from about 0.05 to 1% by weight of anti-microbial agent.

3. Substrate according to claim 1 or 2, **characterized in that** said antimicrobial agent is zinc gluconate.

4. Substrate according to one of claims 1 to 3, **characterized in that** said thermoplastic binder consists of a latex, for example an ethylene/vinyl acetate copolymer (EVA) or of thermally binding fibers.

5. Substrate according to one of claims 1 to 4, **characterized in that** said thermoplastic binder is an ethylene/vinyl acetate copolymer (EVA).

6. Process for the preparation of a substrate according to one of claims 1 to 5, **characterized in that** said antimicrobial agent is incorporated into said substrate by spraying a liquid mixture comprising said thermoplastic binder and said anti-microbial agent onto the substrate or by impregnation or coating said substrate with said mixture.

7. Use of a substrate as defined in one of claims 1 to 5 or as obtained by the process of claim 6 for the manufacture of a sanitary article, a hygiene article or an article for food packaging.

8. Use according to claim 7, **characterized in that** said substrate is used for the manufacture of an impregnated diaper or an absorbent paper.

9. Use according to claim 7, **characterized in that** said substrate is an article for feminine hygiene, such as an absorbent pad or for babies, such as an impregnated diaper.

10. Use according to claim 7, **characterized in that** said substrate is used in the manufacture of a food packaging, namely as an absorbent paper for meat trays.

**Patentansprüche**

1. Vliesartiges Substrat auf der Basis von Papierfasern, das mittels Trockenverfahren erhalten wird, **dadurch gekennzeichnet, daß** es ein thermoplastisches Bindemittel, das den Fasern ermöglicht, sich miteinander zu verbinden, sowie Zink-, Silber- oder Kupfergluconat als antimikrobielles Mittel umfaßt.

2. Substrat nach Anspruch 1, **dadurch gekennzeichnet, daß** es etwa 0,01 bis 10, vorzugsweise etwa 0,05 bis 1 Massenprozent antimikrobielles Mittel aufweist.

3. Substrat nach Anspruch 1 oder 2, **dadurch gekennzeichnet, daß** das antimikrobielle Mittel Zinkgluconat ist.

4. Substrat nach einem der Ansprüche 1 bis 3, **dadurch gekennzeichnet, daß** das thermoplastische Bindemittel von Latex, beispielsweise einem Ethylen-Vinylacetat-Copolymer (EVA), oder von sich unter Wärme verbindenden Fasern gebildet ist.

5. Substrat nach einem der Ansprüche 1 bis 4, **dadurch gekennzeichnet, daß** das thermoplastische Bindemittel ein Ethylen-Vinylacetat-Copolymer (EVA ) ist.

6. Verfahren zur Herstellung eines Substrats nach einem der Ansprüche 1 bis 5, **dadurch gekennzeichnet, daß** das antimikrobielle Mittel **dadurch** in das Substrat eingebettet ist, daß ein das thermoplastische Bindemittel und das antimikrobielle Mittel enthaltendes flüssiges Gemisch auf das Substrat aufgesprüht wird oder daß das Substrat mit dem Gemisch imprägniert oder überzogen wird.

7. Verwendung eines Substrats, wie es in einem der Ansprüche 1 bis 5 definiert ist oder wie es mittels des Verfahrens des Anspruchs 6 erhalten wird, für die Herstellung eines Gesundheitsartikels, eines Hygieneartikels oder eines Artikels für die Lebensmittelverpackung.

8. Verwendung nach Anspruch 7, **dadurch gekennzeichnet, daß** das Substrat für die Herstellung eines imprägnierten Tuchs oder eines Saugpapiers verwendet wird.

9. Verwendung nach Anspruch 7, **dadurch gekennzeichnet, daß** das Substrat ein Artikel für die Frauenhygiene, wie zum Beispiel eine Saugeinlage, oder für Babys, wie zum Beispiel ein imprägniertes Tuch ist.

10. Verwendung nach Anspruch 7, **dadurch gekennzeichnet, daß** das Substrat für die Herstellung einer Lebensmittelverpackung, insbesondere als Saugpapier für Fleischwarenschalen verwendet wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 113254 B
- EP 431002 B

- WO 0132138 A